(19) Europäisches Patentamt
European Patent Office
Office européen des brevets

(11) Veröffentlichungsnummer: **0 127 826**
**B1**

(12) # EUROPÄISCHE PATENTSCHRIFT

(45) Veröffentlichungstag der Patentschrift: **04.07.90**

(21) Anmeldenummer: **84105726.8**

(22) Anmeldetag: **19.05.84**

(51) Int. Cl.[5]: **C 07 D 211/90,**
C 07 D 401/12, A 61 K 31/44

(54) **1,4-Dihydropyridine, Verfahren zu ihrer Herstellung sowie ihre Verwendung in Arzneimitteln.**

(30) Priorität: **01.06.83 DE 3319956**

(43) Veröffentlichungstag der Anmeldung:
**12.12.84 Patentblatt 84/50**

(45) Bekanntmachung des Hinweises auf die Patenterteilung:
**04.07.90 Patentblatt 90/27**

(84) Benannte Vertragsstaaten:
**AT BE CH DE FR GB IT LI NL SE**

(56) Entgegenhaltungen:
**EP-A-0 002 208**
**EP-A-0 071 819**
**DE-A-2 228 377**
**DE-A-2 629 892**

**Die Akte enthält technische Angaben, die nach dem Eingang der Anmeldung eingereicht wurden und die nicht in dieser Patentschrift enthalten sind.**

(73) Patentinhaber: **BAYER AG**
**D-5090 Leverkusen 1 Bayerwerk (DE)**

(72) Erfinder: **Stoltefuss, Jürgen, Dipl.-Ing.**
**Parkstrasse 20**
**D-5657 Haan (DE)**
Erfinder: **Franckowiak, Gerhard, Dr.**
**Henselweg 10**
**D-5600 Wuppertal 1 (DE)**
Erfinder: **Böshagen, Horst, Dr.**
**Wiesenstrasse 4**
**D-5657 Haan (DE)**
Erfinder: **Goldmann, Siegfried, Dr.**
**Kuckuckstrasse 41**
**D-5600 Wuppertal 2 (DE)**
Erfinder: **Schramm, Matthias, Dr.**
**Paffrather Strasse 38**
**D-5000 Köln 80 (DE)**
Erfinder: **Thomas, Günter, Dr.**
**Claudiusweg 9**
**D-5600 Wuppertal 1 (DE)**
Erfinder: **Gross, Rainer, Dr.**
**Platzhoffstrasse 23**
**D-5600 Wuppertal 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**EP 0 127 826 B1**

**Beschreibung**

Die vorliegende Erfindung betrifft neue 1,4-Dihydropyridine, Verfahren zu ihrer Herstellung sowie ihre Verwendung in Arzneimitteln, insbesondere in kreislaufbeeinflussenden Arzneimitteln mit positiv inotroper Wirkung.

Es ist bereits bekannt geworden, daß 1,4-Dihydropyridine gefäßerweiternde Eigenschaften besitzen und als Coronarmittel und Antihypertensiva verwendet werden können (vgl. britisches Patent 1 173 062; britisches Patent 1 358 951; DE—OS 2 629 892 und DE—OS 2 752 820). Weiterhin ist bekannt, daß 1,4-Dihydropyridine als Calciumantagonisten eine Hemmung der Kontraktionskraft von glatten und kardialen Muskeln bewirken und zur Behandlung von Coronar- und Gefäßerkrankungen eingesetzt werden können (vgl. A. Fleckenstein, Ann. Rev. Pharmacol. Toxicol. *17*, 149—166 (1977)).

In DE—A 2 228 377 werden bereits Dihydropyridinamide beschrieben, die in 3- und 5-Position jeweils eine Carbonylgruppe tragen und calciumantagonistische Wirkungen zeigen.

In EP—A 2 208 werden 5-Nitro-dihydropyridine beschrieben, die in 3-Position keine Amidgruppe tragen. Als pharmakologische Wirkung ist die klassische calciumantagonistische Wirkung angegeben.

In EP—A 71 819 werden erstmals Dihydropyridine mit calciumagonistischer Wirkung beschrieben. Die sehr breit gefaßte Definition dieser Anmeldung umfaßt auch einige erfindungsgemäße Verbindungen, ohne sie jedoch namentlich zu nennen. Die ausgewählten Verbindungen der vorliegenden Erfindung sind als solche in EP—A 71 819 nicht beschrieben.

In der nachpublizierten DE—A 3 348 281 (deutsche Priorität 30. April 1983) ist die Verbindung 1,4-Dihydro-2,6-dimethyl-3-nitro-4-[2-(4-methyl-benzyloxy)-phenyl]-5-carbonsäure-cyclopropylamid beschrieben. Im Hinblick auf diese nur in Deutschland existente Anmeldung wird für den Vertragsstatt Deutschland ein gesonderter Satz Patentansprüche mit entsprechendem Disclaimer aufgestellt.

Bei Kenntnis der Eigenschaften dieser bekannten Dihydropyridine war es nicht vorhersehbar, daß die erfindungsgemäßen Verbindungen aus dieser Stoffklasse keine kontraktionshemmende, sondern eine kontraktionskraftverstärkende, am Herzmuskel positiv inotrope Wirkung besitzen.

Die vorliegende Anmeldung betrifft neue 1,4-Dihydropyridin-diamide der allgemeinen Formel I

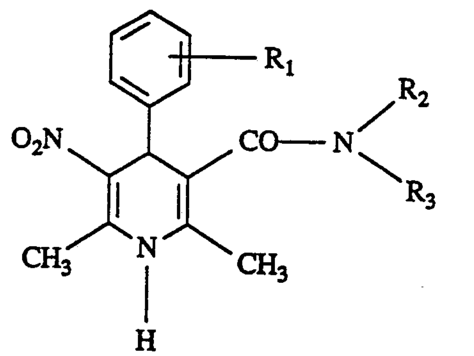

(I)

in welcher

$R_1$ Halogen, Trifluormethyl, Nitro, Wasserstoff oder eine der Gruppen

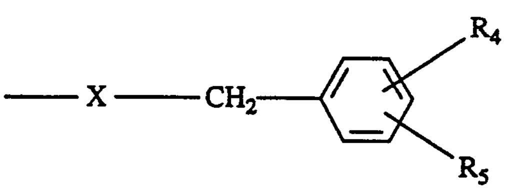

oder

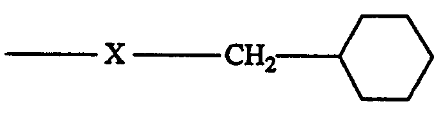

in denen X Sauerstoff oder Schwefel darstellt, wobei $R_4$ und $R_5$ gleich oder verschieden sein können und Wasserstoff, Halogen oder $C_1$—$C_4$-Alkyl bedeuten,

$R_2$ Wasserstoff oder $C_1$—$C_4$-Alkyl darstellt und

$R_3$ Wasserstoff, $C_2$—$C_4$-Alkenyl, $C_1$—$C_4$-Alkyl gegebenenfalls ein oder mehrfach substituiert durch Hydroxy, $C_3$—$C_6$-Cycloalkyl, Di-$C_1$—$C_4$-alkylamino-$C_1$—$C_4$-alkyl, Phenyl, $C_1$—$C_4$-Alkyl substituiert durch Phenyl, oder einen Kohlenhydratrest bedeutet,

sowie ihre pharmazeutisch unbedenklichen Additionssalze.

Als Salze seien beispielhaft genannt: Hydrochloride, Hydrogensulfate, Acetate, Maleate, Benzoate, Citronate, Tartrate oder Lactate.

Als Kohlenhydratrest kommen vor allem in Betracht: Monosaccharidreste in der Furanose- und/oder Pyranoseform sowie Derivate des 1-Desoxynojirimycins. Besonders bevorzugte Reste sind 2-Desoxy-D-glucose, Glucosyl, der 1-Desoxynojirimycin-1-methylen-Rest, 1-Desoxynojirimycin-5-methylen-Rest und der N-(1-Desoxynojirimycinylpropylen)-Rest.

Die erfindungsgemäßen Dihydropyridine der allgemeinen Formel (I) können hergestellt werden, indem man

A) Aminocrotonsäureamide der allgemeinen Formel (II)

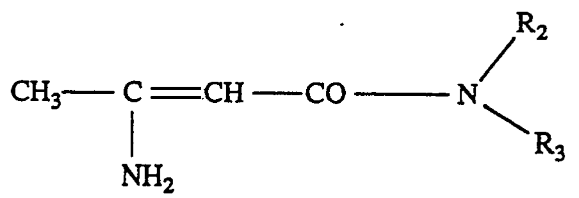

(II)

in welcher $R_2$ und $R_3$ die oben angegebene Bedeutung haben, mit Aldehyden der allgemeinen Formel (III)

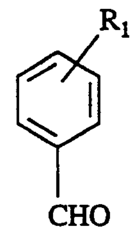

(III)

in welcher $R_1$ die oben angegebene Bedeutung hat und Nitroaceton

$$CH_3-CO-CH_2-NO_2$$

umsetzt oder

B) Benzaldehyde der Formel III und Acetessigsäureamide der Formel (IV)

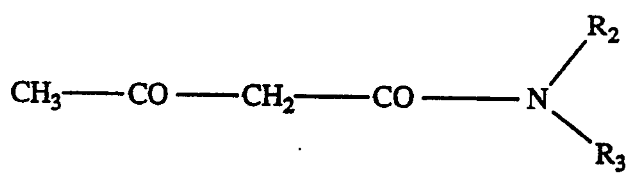

(IV)

in welcher $R_2$ und $R_3$ die oben angegebene Bedeutung haben bzw. deren Knoevenagel-Kondensationsprodukte der Formel (V)

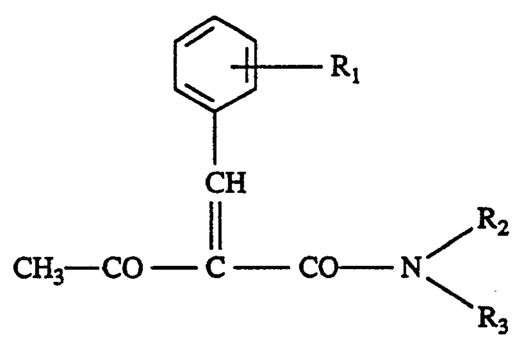

(V)

und einer Additionsverbindung aus Nitroaceton und Ammoniak

$$CH_3-CO-CH_2-NO_2 \cdot NH_3$$

umsetzt oder,

C) indem man Dihydropyridine der Formel (VI)

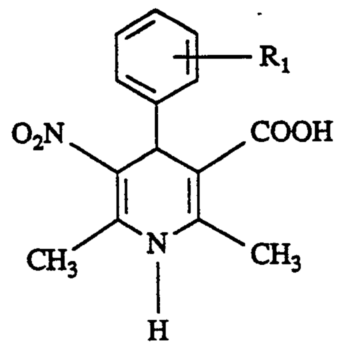

(VI)

in welcher $R_1$ die oben angegebene Bedeutung hat, nach an sich bekannten Methoden mit einem Amin umsetzt oder in ein reaktives Säurederivat überführt und dieses dann mit Aminen der allgemeinen Formel (VII)

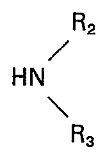

(VII)

in welcher $R_2$ und $R_3$ die oben angegebene Bedeutung haben, umsetzt.

Als reaktives Säurederivat sei beispielhaft genannt: aktivierter Ester, Hydroxysuccinimidester, Säureimidazolide, gemischte Anhydride, Umsetzung mit Dicyclohexylcarbodiimid.

Setzt man z.B. nach Verfahrensvariante A) β-Aminocrotonsäuredimethylamid und 2-Chlorbenzaldehyd mit Nitroaceton um, so läßt sich die Reaktion durch folgendes Formelschema wiedergeben:

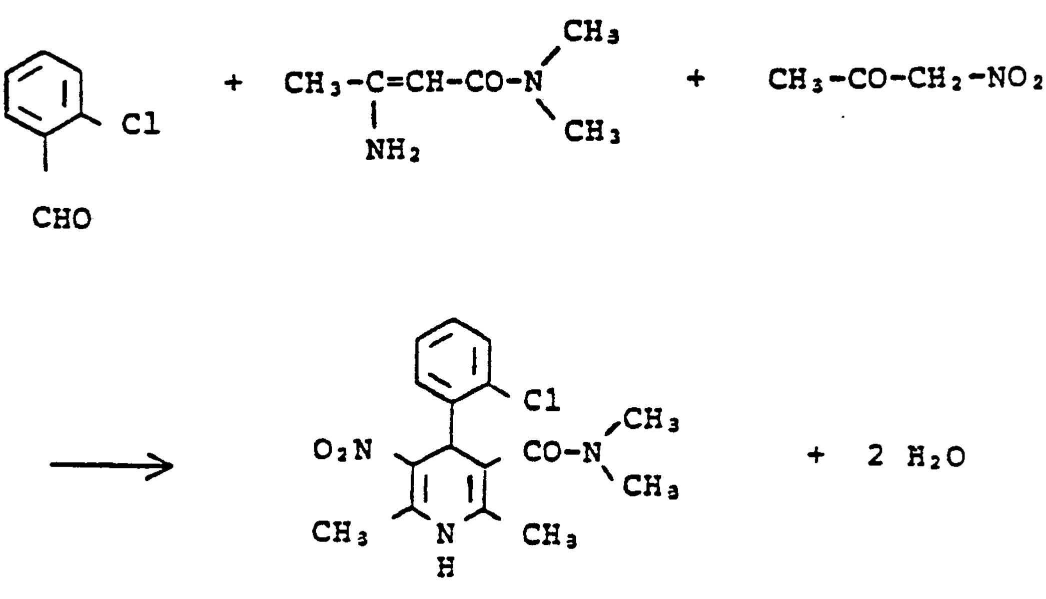

Setzt man z.B. nach Verfahrensvariante B) 2-Trifluorbenzyliden-acetessigsäurediethylamid mit Nitroaceton/Ammoniak um, so läßt sich die Reaktion durch folgendes Formelschema wiedergeben:

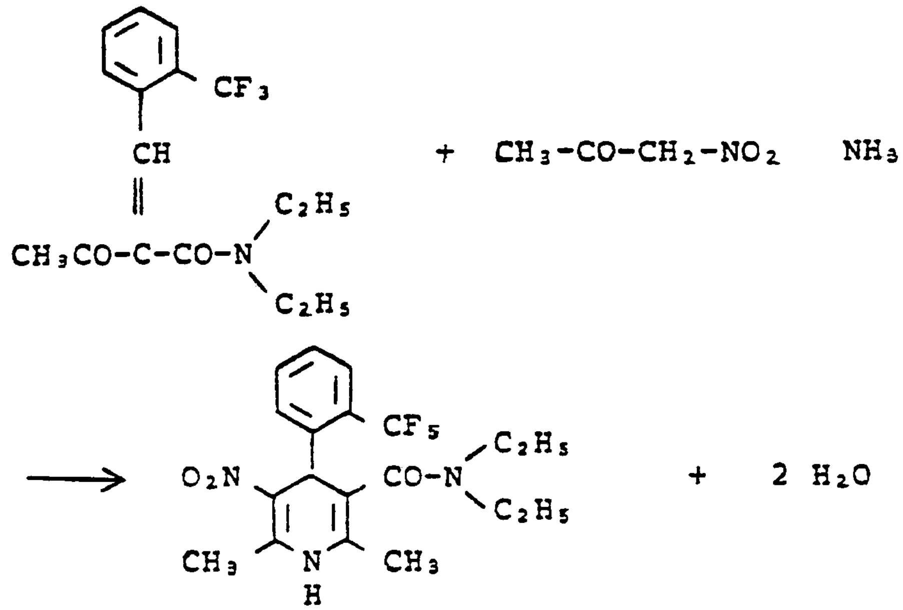

Setzt man z.B. nach Verfahrensvariante C) 2,6-Dimethyl-4-(2-(4-methylbenzyloxy)-phenyl)-3-nitro-1,4-dihydropyridin-5-carbonsäure mit Carbonyldiimidazol um und läßt das erhaltene Imidazolid mit Cyclopropylamin reagieren, so läßt sich die Reaktion durch das folgende Formelschema wiedergeben:

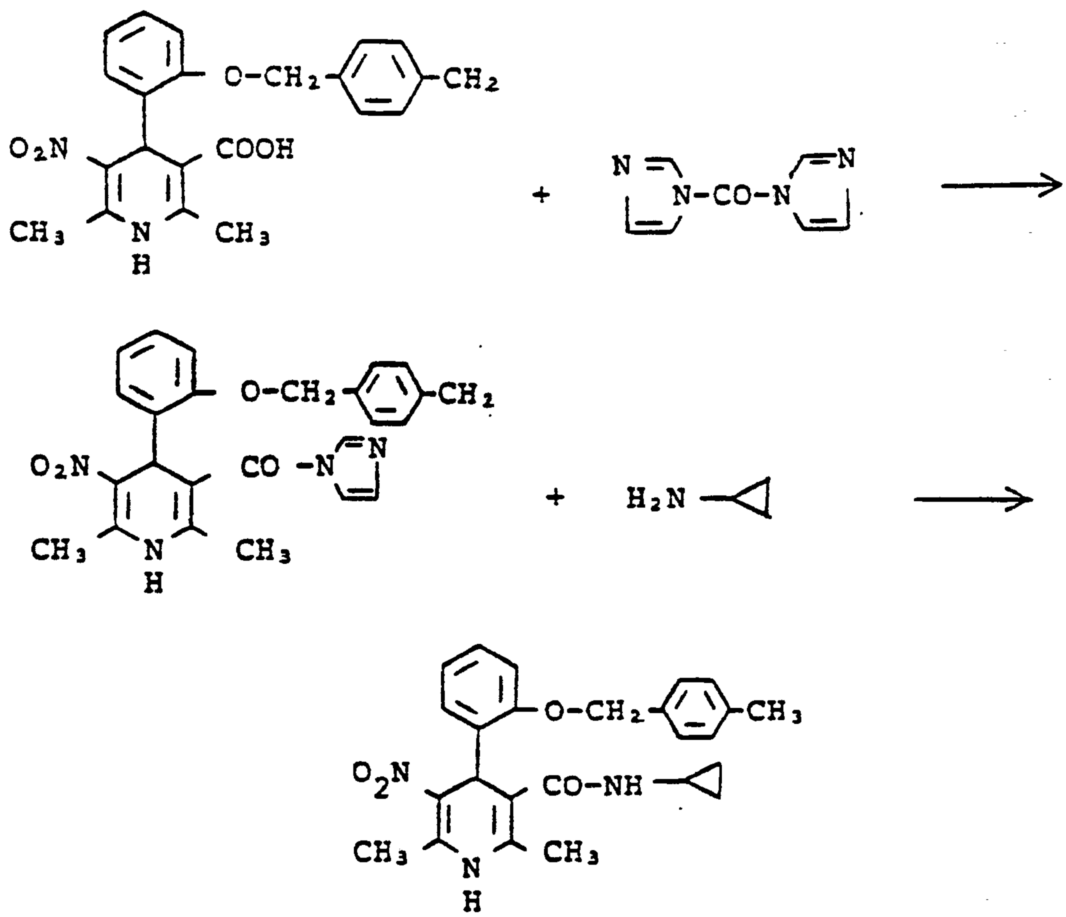

Für alle Verfahrensvarianten A, B und C kommen als Verdünnungsmittel alle inerten organischen Lösungsmittel in Frage. Hierzu gehören vorzugsweise Alkohole wie Ethanol, Methanol, Isopropanol, Ether wie Dioxan, Diethylether, Tetrahydrofuran, Glykolmonomethylether, Glykoldimethylether oder Eisessig, Dimethylformamid, Dimethylsulfoxid, Acetonitril, Pyridin und Hexamethylphosphorsäuretriamid.

Die Reaktionstemperaturen können in einam größeren Bereich variiert werden. Im allgemeinen

arbeitet man zwischen 20 und 150°C, vorzugsweise jedoch bei der Siedetemperatur des jeweiligen Lösungsmittels.

Die Umsetzung kann bei Normaldruck, aber auch bei erhöhtem Druck durchgeführt werden. Im allgemeinen arbeitet man unter Normaldruck.

Die vorstehenden Herstellungsverfahren sind lediglich zur Verdeutlichung angegeben, und die Herstellung der Verbindungen der Formel (I) ist nicht auf diese Verfahren beschränkt, sondern jede Modifikation dieser Verfahren ist in gleicher Weise für die Herstellung der erfindungsgemäßen Verbindungen anwendbar.

Das Mengenverhältnis der Reaktanden zueinander ist beliebig, im allgemeinen werden äquimolare Mengen eingesetzt. Es hat sich jedoch als zweckmäßig erwiesen, im Verfahren A Nitroaceton im bis zu 5 molaren Überschuß im Verfahren B das Nitroaceton/Ammoniak-Addukt im bis zu 5 molaren Überschuß und im Verfahren C das Amin der Formel VII im Überschuß bis zu 10 Molen einzusetzen.

Je nach der Wahl der Ausgangssubstanzen können die erfindungsgemäßen Verbindungen in stereoisomeren Formen existieren, die sich entweder wie Bild und Spiegelbild (Enantiomere) oder die sich nicht wie Bild und Spiegelbild (Diastereomere) verhalten. Sowohl die Antipoden als auch die Racemformen als auch die Diastereomerengemische sind Gegenstand der vorliegenden Erfindung. Die Racemformen lassen sich ebenso wie die Diastereomeren in bekannter Weise in die stereoisomer einheitlichen Bestandteile trennen (vgl. z.B. E. L. Eliel, Stereochemistry of Carbon Compounds, McGraw Hill, 1962).

Die als Ausgangsstoffe verwendeten Aldehyde (III) sind bekannt oder können nach literaturbekannten Methoden dargestellt werden (vgl. *T. D. Harris and G. P. Roth*, J. org. Chem. *44*, 146 (1979)
Deutsche Offenlegungsschrift 2 165 260
Deutsche Offenlegungsschrift 2 401 665
*Mijano et al.*, Chem. Abstr. 59, (1963), 13 929 c,
*E. Adler und H.-D. Becker*, Chem. Scand. 15, 849 (1961),
*E. P. Papadopoulos, M. Mardin* und *Ch. Issidoridis*,
J. Org. Chem. *31*, 615 (1966), J. Am. chem. Soc. *78*, 2543 (1956)).

Die Aminocrotonsäureamide der allgemeinen Formel (II) sind bereits bekannt oder können nach bekannten Methoden hergestellt werden (siehe Deutsche Offenlegungsschrift 2 228 377).

Die erfindungsgemäß verwendbaren Yliden-β-carbonsäureamide der Formel (V) sind bereits bekannt oder können nach bekannten Methoden hergestellt werden [Organic Reactions XV, 204 ff (1967)].

Die erfindungsgemäß verwendbaren Dihydropyridinderivate der Formel (VI) sind zum Teil bekannt oder lassen sich nach bekannten Methoden herstellen (siehe EP 71 819).

Die Amine der Formel (VII) sind zum größten Teil bekannt oder lassen sich nach bekannten Methoden herstellen (vgl. Houben-Weyl, Methoden der organischen Chemie, Band 11/1; Paulsen, Angew. Chemie *78*, 501—566 (1966); Le A 18 389 = EP 947).

Carbonsäuren lassen sich in den meisten Fällen nur schwer mit Aminen direkt zu Carbonsäureamiden umsetzen. Man setzt daher reaktive Carbonsäurederivate, in den einfachsten Fällen Carbonsäurehalogenide oder Carbonsäureanhydride, ein. Die Herstellung von Carbonsäureamiden unter milderen Bedingungen gelingt z.B. durch Umsetzung der Carbonsäure mit Dicyclohexylcarbodiimid und Aminen nach folgendem Schema

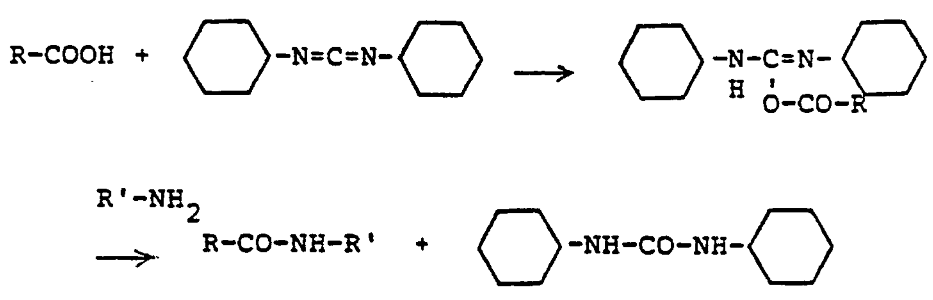


vgl. Fieser and Fieser, Reagents for Organic Synthesis
John Wiley & Sons, Inc. (1967), Seite 231—36.
J. C. Sheehan and G. P. Hess, J. Am. Chem. Soc. *77*, 1067 (1955);
U. Goodman and G. W. Kenner, Adv. in Protein Chem. *12*, 488 (1957); W. A. Bonner and P. I. McNamee, J. Org. Chem. *26*, 2554 (1961).

Weitere aktivierte Carbonsäurederivate sind beispielsweise Carbonsäurehydroxysuccinimidester, Carbonsäureimidazolide
[Fieser and Fieser, Reagents for Organic Synthesis, John Wiley & Sons, Inc. 1967, Seite 114—116; H. A. Staab, Angew. Chemie, Internat. Ed. 1, 351 (1962)]; N,N'-Carbonyldi-s-triazin [Fieser and Fieser, Reagents for Organic Syntheses, John Wiley & Sons, Inc. 1967, Seite 116; H. C. Beyerman and W. U. Van den Brink, Rec. Trav. *80*, 1372 (1961)], ohne vollständig zu sein.

(Siehe auch Methoden zur Amidbildung in: Survey of Organic Synthesis, Calvin A. Buehler and Donald E. Pearson, John Wiley & Sons, Volume 1 (1970) Seite 895 ff und Volume 2 (1977).

Nitroaceton läßt sich nach bekannten Methoden herstellen (vgl. N. Levy u. C. W. Scarfe, J. Chem. Soc. (London) (1946) 1103 C. D. Hurd and M. E. Nilson, J. Org. Chem. *20*, 927 (1955)).

Die neuen Wirkstoffe können in bekannter Weise in die üblichen Formulierungen übergeführt werden, wie Tabletten, Kapseln, Dragees, Pillen, Granulate, Aerosole, Sirupe, Emulsionen, Suspensionen und Lösungen, unter Verwendung inerter, nicht-toxischer, pharmazeutisch geeigneter Trägerstoffe oder Lösungsmittel. Hierbei soll die therapeutisch wirksame Verbindung jeweils in einer Konzentration von etwa 0,5 bis 90 Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Als Hilfsstoffe seien beispielsweise aufgeführt:

Wasser, nicht-toxische organische Lösungsmittel, wie Paraffine (z.B. Erdölfraktionen), pflanzliche Öle (z.B. Erdnuß-/Sesamöl), Alkohole (z.B. Ethylalkohol, Glycerin), Glykole (z.B. Propylenglykol, Polyethylenglykol), feste Trägerstoffe, wie z.B. natürliche Gesteinsmehle (z.B. Kaoline, Tonerden, Talkum, Kreide), synthetische Gesteinsmehle (z.B. hochdisperse Kieselsäure, Silikate), Zucker (z.B. Rohr-, Milch- und Traubenzucker), Emulgiermittel (z.B. Polyoxyethylen-Fettsäure-Ester, Polyoxyethylen-Fettalkohol-Ether, Alkylsulfonate und Arylsulfonate), Dispergiermittel (z.B. Lignin, Sulfitablaugen, Methylcellulose, Stärke und Polyvinylpyrrolidon) und Gleitmittel (z.B. Magnesiumstearat, Talkum, Stearinsäure und Natriumlaurylsulfat).

Die Applikation erfolgt in üblicher Weise, vorzugsweise oral oder parenteral, insbesondere perlingual oder intravenös. Im Falle der oralen Anwendung können Tabletten selbstverständlich außer den genannten Trägerstoffen auch Zusätze, wie Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wäßriger Suspensionen und/oder Elixieren, die für orale Anwendungen gedacht sind, können die Wirkstoffe außer den obengenannten Hilfsstoffen mit verschiedenen Geschmacksaufbesserern oder Farbstoffen versetzt werden.

Für den Fall der parenteralen Anwendung können Lösungen der Wirkstoffe unter Verwendung geeigneter flüssiger Trägermaterialien eingesetzt werden.

Im allgemeinen hat es sich als vorteilhaft erwiesen, bei intravenöser Applikation Mengen von etwa 0,001 bis 1 mg/kg, vorzugsweise etwa 0,01 bis 0,5 mg/kg Körpergewicht zur Erzielung wirksamer Ergebnisse zu verabreichen, und bei oraler Applikation beträgt die Dosierung etwa 0,01 bis 20 mg/kg, vorzugsweise 0,1 bis 10 mg/kg Körpergewicht.

Trotzdem kann es gegebenenfalls erforderlich sein, von den genannten Mengen abzuweichen, und zwar in Abhängigkeit vom Körpergewicht des Versuchstieres bzw. der Art des Applikationsweges, aber auch aufgrund der Tierart und deren individuellem Verhalten gegenüber dem Medikament bzw. deren Art von dessen Formulierung und dem zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehrere Einzelgaben über den Tag zu verteilen. Für die Applikation in der Humanmedizin ist der gleiche Dosierungsspielraum vorgesehen. Sinngemäß gelten hierbei auch die obigen Ausführungen.

Die erfindungsgemäßen Verbindungen haben eine positive inotrope Wirkung und zeigen somit ein nicht vorhersehbares und wertvolles pharmakologisches Wirkungsspektrum. Sie können als Cardiotonika zur Verbesserung der Herzkontraktilität dienen. Darüber hinaus können sie als Antihypotonika, zur Senkung von Blutzucker, zur Abschwellung von Schleimhäuten und zur Beeinflussung des Salz- und Flüssigkeitshaushaltes eingesetzt werden.

Die positiv inotrope Wirkung der erfindungsgemäßen Verbindungen der Formel (I) wird in folgender Versuchsanordnung bestimmt:

Die linken Vorhöfe von Meerschweinchenherzen werden isoliert und in ein thermostatisiertes Organbad gehängt, welches eine isotonische Mineralsalzlösung, die dem Ionenmilieu und dem pH-Wert von Körperflüssigkeiten angepaßt ist, mit geeigneten Nährstoffen enthält. Dieses Organbad wird mit einem Gasgemisch bestehend aus Sauerstoff und Kohlendioxid begast, wobei der Kohlendioxidgehalt so bemessen ist, daß der pH-Wert des Organbades konstant bleibt. Die linken Vorhöfe werden in das Organbad eingespannt, die Spannung wird mittels eines Kraftaufnehmers registriert, wobei ein bestimmter Grundtonus eingestellt wird. Anschließend werden die linken Vorhöfe kontinuierlich in bestimmten Abständen elektrisch gereizt und die dabei erfolgenden Kontraktionen registriert. Nach Zugabe des Wirkstoffs werden die Kontraktionen weiterhin registriert. Eine Kontraktionsverstärkung um mindestens 25% gilt als signifikante positivinotrope Wirkung.

So werden zum Beispiel die Kontraktionen des mit 1 Hz elektrisch gereizten linken Meerschweinchenvorhofs durch $10^{-6}$ g/ml der Verbindung aus Beispiel 8 um 38%, durch die Verbindung aus Beispiel 9 um 43%, und durch die Verbindung aus Beispiel 12 um 95%, ebenfalls jeweils bei einer Konzentration der jeweiligen Verbindung von $10^{-6}$ g/ml, verstärkt.

## HERSTELLUNGSBEISPIELE

### Beispiel 1

1,4-Dihydro-2,6-dimethyl-3-nitro-4-(2-benzyloxy-phenyl)-5-carbonsäure-dimethylamid

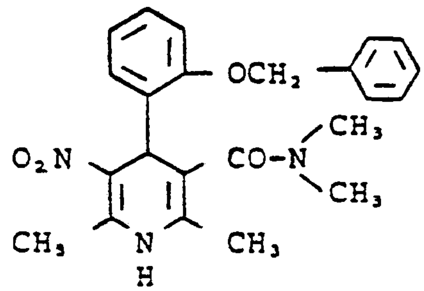

*Verfahrensvariante A*

6.36 g (30 mmol) 2-Benzyloxy-benzaldehyd werden mit 4,8 g (30 mmol) β-Aminocrotonsäuredimethylamid und 6,2 g (60 mmol) Nitroaceton in 45 ml Ethanol 2 Stunden zum Rückfluß erhitzt. Es wird abgekühlt und eingeengt. Der Eindampfrückstand wird in Essigester aufgenommen, es wird mit Wasser, verdünnter Natriumhydrogencarbonatlösung und wieder Wasser gewaschen, getrocknet und eingeengt, wobei Kristallisation eintritt. Es wird mit Ethanol verrührt, abgesaugt und mit Ethanol gewaschen. Man erhält 1,6 g eines gelbgefärbten Produkts vom Schmelzpunkt 204—206°C unter Zersetzung.

### Beispiel 2

1,4-Dihydro-2,6-dimethyl-3-nitro-4-[2-(4-methylbenzyloxy)phenyl]-5-carbonsäure-cyclopropylamid

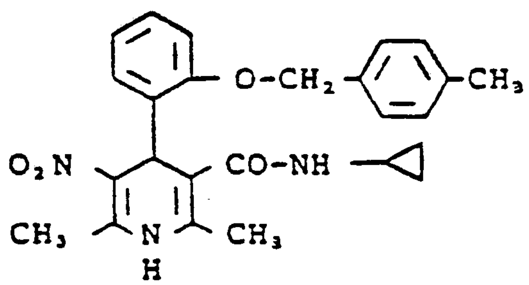

*Verfahrensvariante C*

1. Herstellung von 1,4-Dihydro-2,6-dimethyl-3-nitro-4-[2-(4-methylbenzyloxy)-phenyl]-5-carbonsäureimidazolid 26 g (65,9 mmol) 1,4-Dihydro-2,6-dimethyl-3-nitro-4-[2-(4-methylbenzyloxy)-phenyl]-5-carbonsäure vom Schmelzpunkt 202°C unter Zersetzung werden in 400 ml absolutem THF suspendiert und mit 12,9 g (79,6 mmol) Carbonyldiimidazol versetzt, wobei schnell Lösung eintritt. Nach 4 Stunden werden weitere 6 g Carbonyldiimidazol zugegeben. Es wird 18 Stunden gerührt und eingeengt. Der Eindampfrückstand wird in Methylenchlorid aufgenommen, es wird 2 × schnell mit Wasser gewaschen, getrocknet und eingeengt. Der Eindampfrückstand wird durch Zugabe von Acetonitril kristallisiert, abgesaugt und mit Acetonitril gewaschen. Man erhält 21,6 g (73,7% d.Th.) des gelbgefärbten Imidazolids vom Schmelzpunkt 193—194°C.

2. 4 g 1,4-Dihydro-2,6-dimethyl-3-nitro-4-[2-(4-methylbenzyloxy)-phenyl]-5-carbonsäure-imidazolid werden in 40 ml absolutem Tetrahydrofuran mit 10 ml Cyclopropylamin 20 Stunden zum Rückfluß gekocht. Es wird abgekühlt und eingeengt. Der Eindampfrückstand wird in Essigester aufgenommen und mit 1N Salzsäure und Wasser ausgeschüttelt. Die organische Phase wird getrocknet und eingeengt. Der erhaltene Eindampfrückstand wird in wenig heißem Ethanol gelöst. Nach mehrstündigem Stehen werden die erhaltenen Kristalle abgesaugt und mit Ethanol gewaschen. Man erhält 3,2 g 1,4-Dihydro-2,6-dimethyl-3-nitro-4-[2-(4-methylbenzyloxy)-phenyl]-5-carbonsäure-cyclopropylamid vom Schmelzpunkt 198°C.

Die analog Verfahrensvariante C hergestellten Verbindungen sind in folgender Tabelle aufgeführt.

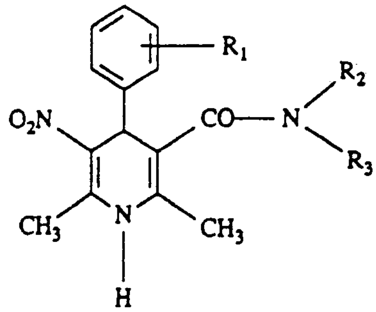

(I)

| Beisp. Nr. | Posi-tion | $R_1$ | $R_2$ | $R_3$ | Schmp.: °C |
|---|---|---|---|---|---|
| 3 | 2 | -Cl | H | 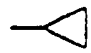 | 183-85 |
| 4 | 3 | Cl | H | $-{}_nC_4H_9$ | 223-24 |
| 5 | 2 | $-OCH_2-$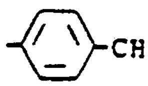$-CH_3$ | H | $-{}_nC_4H_9$ | 203-04 |
| 6 | 2 | $-O-CH_2-$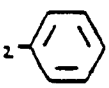 | H | 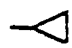 | 221 |
| 7 | 2 | $-O-CH_2-$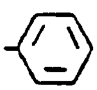 | H | $-CH_2-CH_2-OH$ | 192-93 |
| 8 | 2 | $-O-CH_2-$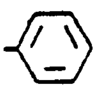 | H | $-CH_2-CH=CH_2$ | 186 |
| 9 | 2 | $-O-CH_2-$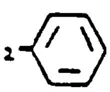 | H | $CH_3$<br>$-C-CH_3$<br>$CH_3$ | 160 |
| 10 | 2 | $-O-CH_2-$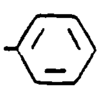 | H | H | 230-31 |
| 11 | 2 | $-OCH_2-$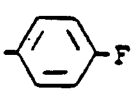$-F$ | H | H | 142 Z |
| 12 | 2 | $-O-CH_2-$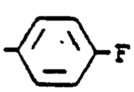$-F$ | H | 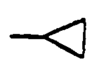 | ab 124 |

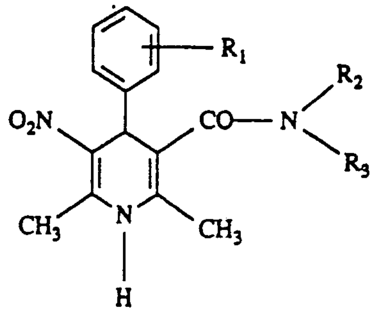

(I)

| Beisp. Nr. | Posi-tion | $R_1$ | $R_2$ | $R_3$ | Schmp.: °C |
|---|---|---|---|---|---|
| 13 | 2 | 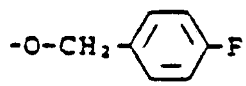 | H | $-CH_2-CH_2-OH$ | 129-30 |
| 14 | 2 | 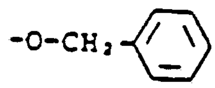 | H | $-CH_3$ | 229 Z |
| 15 | 2 | 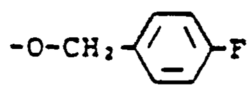 | H | $CH_3$ | 203 |
| 16 | 2 | 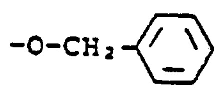 | H | $C_2H_5$ | 211-12 |
| 17 | 2 | 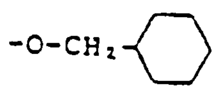 | H | 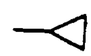 | 191-93 |
| 18 | 2 | 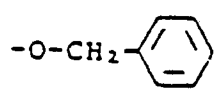 | H | 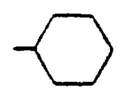 | 210-11 |
| 19 | 2 | 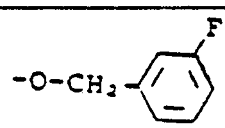 | H | 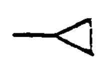 | 221-23 |
| 20 | 2 | 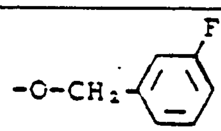 | H | H | 138 Z x 1 $H_2O$ |
| 21 | 2 | 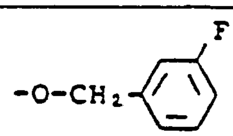 | H | $CH_3$ | 235 |
| 22 | 2 | 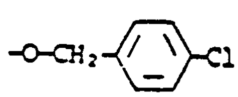 | H | H | 171-75 |

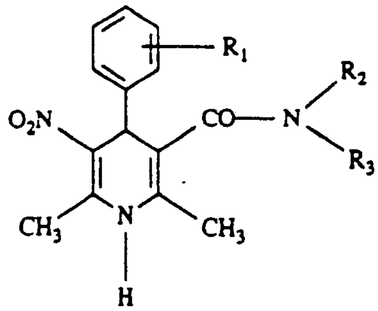

(I)

| Beisp. Nr. | Position | $R_1$ | $R_2$ | $R_3$ | Schmp.: °C |
|---|---|---|---|---|---|
| 23 | 2 | 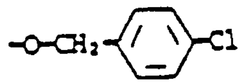 | H | $CH_3$ | 237 |
| 24 | 2 | 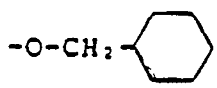 | H | H | 218-20 |
| 25 | 2 | 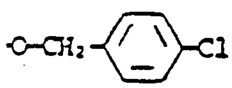 | H | 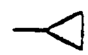 | 147 |
| 26 | 2 | $CF_3$ | H | 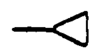 | 253 Z |
| 27 | 3 | $NO_2$ | H | 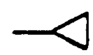 | ab 120 Z |
| 28 | 2 | 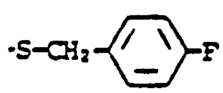 | H | 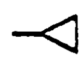 | 167-70 |
| 29 | 2 | 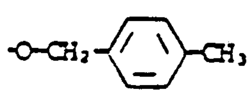 | H | H | 135 Z |
| 30 | 2 | 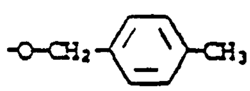 | H | 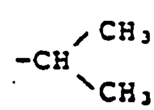 | 222-24 |
| 31 | 2 | 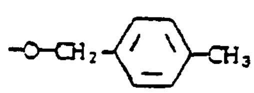 | H | $-CH_2-CH_2-OH$ | 230-32 |
| 32 | 2 | 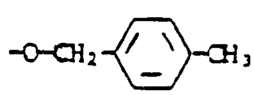 | H | 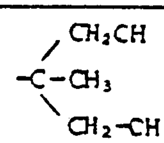 | 220-22 |

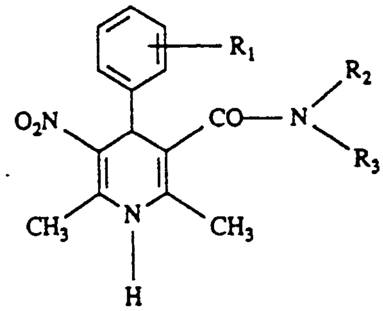

(I)

| Beisp. Nr. | Posi-tion | $R_1$ | $R_2$ | $R_3$ | Schmp.: °C |
|---|---|---|---|---|---|
| 33 | 2 | 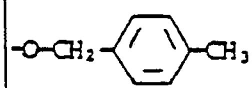 | H | 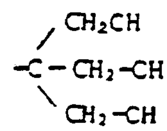 | 224 |
| 34 | 2 | 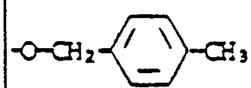 | H | 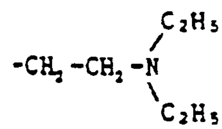 | 156-158 |
| 35 | 2 | 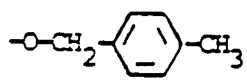 | H | 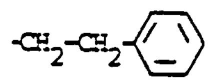 | 210-12 |
| 36 | 2 | 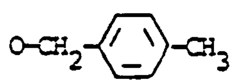 | H | 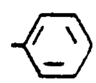 | 259-60 |
| 37 | | H | $CH_3$ | $CH_3$ | 244 |
| 38 | | H | H | 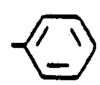 | 251-54 |
| 39 | | H | H | 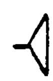 | 242 |
| 40 | 3 | -F | H | 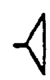 | 250 |
| 41 | 2 | -F | H | 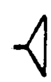 | 224-26 |
| 42 | 2 | -F | H | $-CH_2-CH=CH_2$ | 200 |

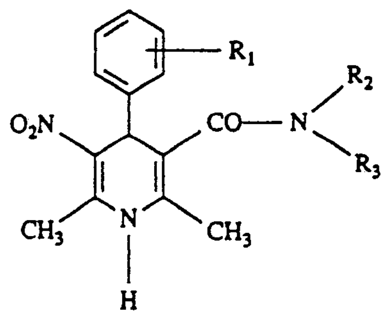

(I)

| Beisp. Nr. | Position | $R_1$ | $R_2$ | $R_3$ | Schmp.: °C |
|---|---|---|---|---|---|
| 43 | 2 | 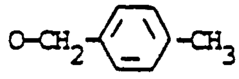 | H | $-CH_2-CH=CH_2$ | 232 |
| 44 | 2 | 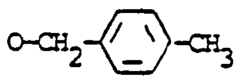 | H | 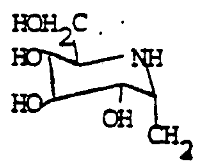 | 149-51 |
| 45 | 2 | 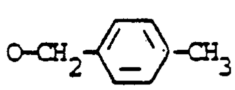 | H | 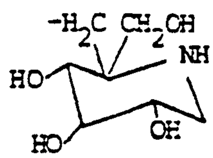 | 242 |
| 46 | 3 | $-NO_2$ | H | 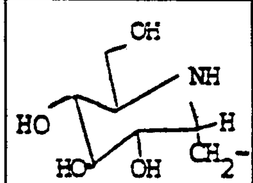 | 160°C |

Analog Verfahrensvariante A wurden hergestellt:

Beispiel 47

1,4-Dihydro-2,6-dimethyl-3-nitro-4-(2-trifluormethylphenyl)-5-carbonsäureanilid vom Schmp.; 232°C.

Beispiel 48

1,4-Dihydro-2,6-dimethyl-3-nitro-4-(3-nitrophenyl)-5-carbonsäurediethylamid vom Schmp.: 197°C.

Beispiel 49

1,4-Dihydro-2,6-dimethyl-3-nitro-4-(2-trifluormethylphenyl)-5-carbonsäuredimethylamid vom Schmp.; 218°C.

Beispiel 50 (Verfahrensvariante B)

1,4-Dihydro-2,6-dimethyl-3-nitro-4-(2-benzyloxy-phenyl)-5-carbonsäure-dimethylamid

3.6 g (30 mmol) Nitroaceton-Ammoniak-Addukt (hergestellt aus Nitroaceton mit einer ethanolischen Ammoniaklösung) werden mit 2.6 g (20 mmol) Acetessigsäuredimethylamid und 4.24 g (29 mmol) 2-Benzyloxybenzaldehyd in 30 ml Ethanol 1 Stunde bei 60°C und anschließend 3 Stunden bei Siedetemperatur gerührt. Es wird abgekühlt und eingeengt. Der erhaltene Eindampfrückstand wird über eine 25 cm lange Säule von 5 cm Durchmesser getrennt, die als stationäre Phase Kieselgel 60 (Merck),

0.04—0.063 mm und als mobile Phase Toluol/Essigester im Volumenverhältnis 10:1 enthält. Die mobile Phase wird während des Trennvorganges stufenweise bis zu Toluol/Essigester im Volumenverhältnis 1:1 verändert.

Die Fraktionen mit dem nahezu reinen Produkt werden zusammengefaßt und eingeengt. Man erhält 234 mg einer nahezu reinen Substanz, die identisch ist mit der im Herstellungsbeispiel 1 beschriebenen Verbindung.

Nach Verfahrensvariante C wurden weiterhin hergestellt:

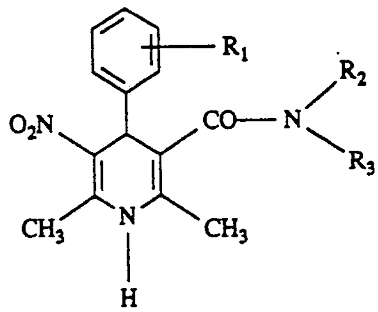

(I)

| Beisp. Nr. | Position | $R_1$ | $R_2$ | $R_3$ | Schmp.: °C |
|---|---|---|---|---|---|
| 51 | 2 | 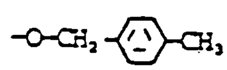 | H | 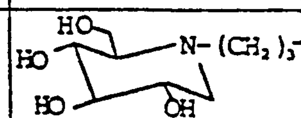 | ab 130 Rf-Wert= 0.325 |
| 52 | 2 | 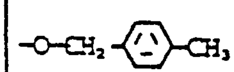 | H | $-CH_2-CH_2-CH_2-OH$ | 211 |
| 53 | 2 | 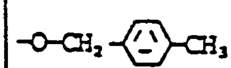 | H | 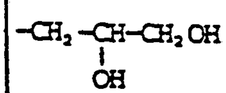 | 209-10 Z |
| 54 | 2 | $-O-CH_2-$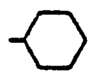 | H | 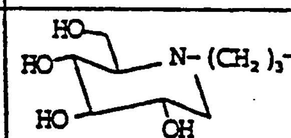 | Rf-Wert= 0.385 |
| 55 | 2 | 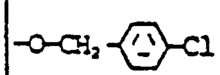 | H | 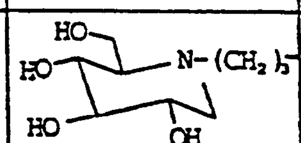 | Rf-Wert= 0.35 |
| 56 | 2 | $-O-CH_2-$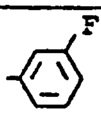 | H | 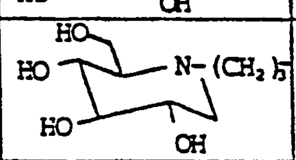 | Rf-Wert= 0.305 |
| 57 | 2 | $-O-CH_2-$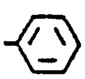 | H | 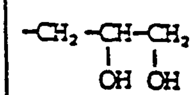 | 132° |

Rf-Wert DC-Fertigplatten (Merck) Kieselgel 60 F 254

Laufmittel: Chloroform/Methanol im Volumenverhältnis 2:

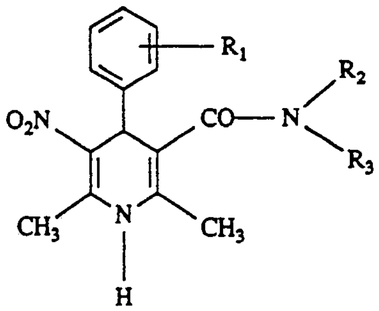

(I)

| Beisp. Nr. | Position | $R_1$ | $R_2$ | $R_3$ | Schmp.: °C |
|---|---|---|---|---|---|
| 58 | 2 | $-O-CH_2-$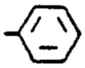 | H | $-CH_2-CH_2-CH_2-OH$ | 159-161° |
| .59 | 2 | $-O-CH_2-$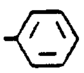 | H | $-CH_2-CH_2-CH_3$ | 102° |

**Patentansprüche für die Vertragsstaaten: BE CH FR GB IT LI NL SE AT**

1. 1,4-Dihydropyridinamide der allgemeinen Formel I

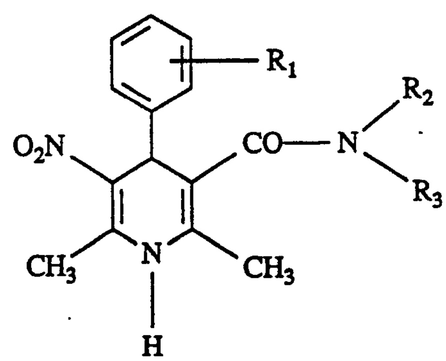

(I)

in welcher

$R_1$ Halogen, Trifluormethyl, Nitro, Wasserstoff oder eine der Gruppen

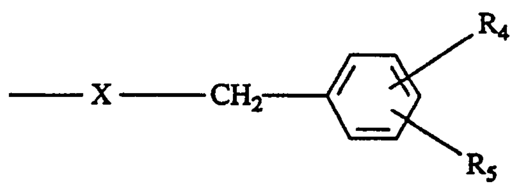

oder

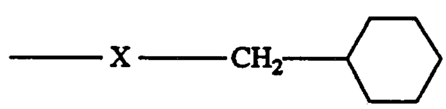

in denen X Sauerstoff oder Schwefel darstellt, wobei $R_4$ und $R_5$ gleich oder verschieden sein können und Wasserstoff, Halogen oder $C_1$—$C_4$-Alkyl bedeuten,

$R_2$ Wasserstoff oder $C_1$—$C_4$-Alkyl darstellt und

$R_3$ Wasserstoff, $C_2$—$C_4$-Alkenyl, $C_1$—$C_4$-Alkyl gegebenenfalls ein oder mehrfach substituiert durch

Hydroxy, $C_3$—$C_6$-Cycloalkyl, Di-$C_1$—$C_4$-alkylamino-$C_1$—$C_4$-alkyl, Phenyl, $C_1$—$C_4$-Alkyl substituiert durch Phenyl, oder einen Kohlenhydratrest bedeutet,
sowie ihre pharmazeutisch unbedenklichen Additionssalze.

2. Verbindungen gemäß Anspruch 1 zur Bekämpfung von Kreislauferkrankungen.

3. Verbindungen gemäß Anspruch 1 zur Verbesserung der Herzkontraktilität, zur Erhöhung des Blutdruckes, zur Senkung des Blutzuckers, zur Abschwellung von Schleimhäuten und/oder zur Beeinflussung des Salz- und Flüssigkeitshaushaltes.

4. Verfahren zur Herstellung von 1,4-Dihydropyridinamiden der allgemeinen Formel I

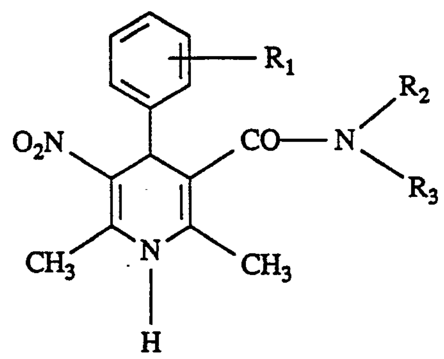

(I)

in welcher
$R_1$ Halogen, Trifluormethyl, Nitro, Wasserstoff oder eine der Gruppen

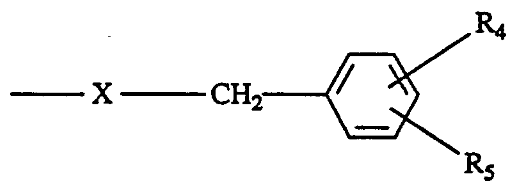

oder

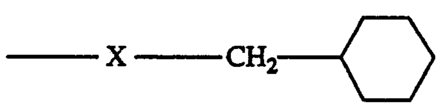

in denen X Sauerstoff oder Schwefel darstellt, wobei $R_4$ und $R_5$ gleich oder verschieden sein können und Wasserstoff, Halogen oder $C_1$—$C_4$-Alkyl bedeuten,
$R_2$ Wasserstoff oder $C_1$—$C_4$-Alkyl darstellt und
$R_3$ Wasserstoff, $C_2$—$C_4$-Alkenyl, $C_1$—$C_4$-Alkyl gegebenenfalls ein oder mehrfach substituiert durch Hydroxy, $C_3$—$C_6$-Cycloalkyl, Di-$C_1$—$C_4$-alkylamino-$C_1$—$C_4$-alkyl, Phenyl, $C_1$—$C_4$-Alkyl substituiert durch Phenyl, oder einen Kohlenhydratrest bedeutet,
dadurch gekennzeichnet, daß man
A) Aminocrotonsäureamide der allgemeinen Formel (II)

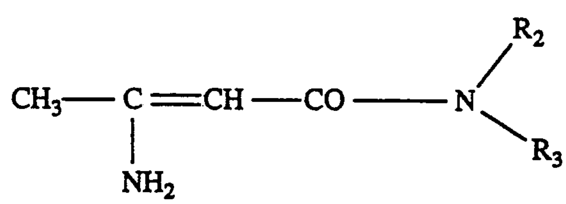

(II)

in welcher $R_2$ und $R_3$ die oben angegebene Bedeutung haben, mit Aldehyden der allgemeinen Formel (III)

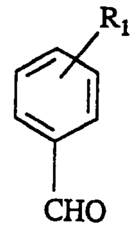

(III)

in welcher $R_1$ die oben angegebene Bedeutung hat und Nitroaceton

$$CH_3—CO—CH_2—NO_2$$

umsetzt oder

B) Benzaldehyde der Formel III und Acetessigsäureamide der Formel (IV)

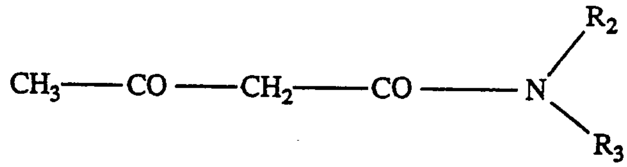

(IV)

in welcher $R_2$ und $R_3$ die oben angegebene Bedeutung haben bzw. deren Knoevenagel-Kondensationsprodukte der Formel (V)

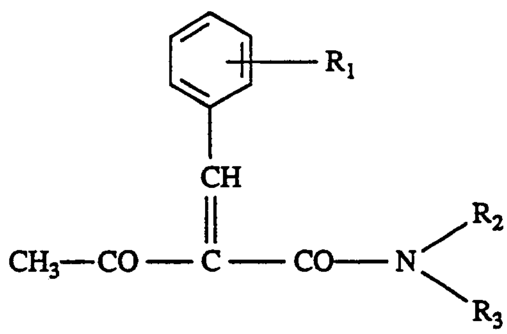

(V)

und einer Additionsverbindung aus Nitroaceton und Ammoniak

$$CH_3—CO—CH_2—NO_2 \cdot NH_3$$

umsetzt oder,

C) Dihydropyridine der Formel (VI)

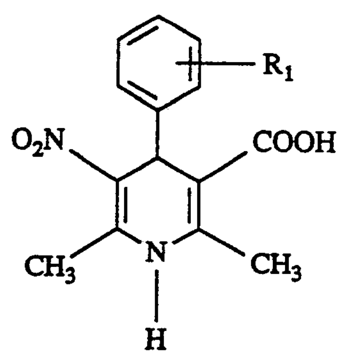

(VI)

in welcher $R_1$ die oben angegebene Bedeutung hat, nach an sich bekannten Methoden mit einem Amin

umsetzt oder in ein reaktives Säurederivat überführt und dieses dann mit Aminen der allgemeinen Formel (VII)

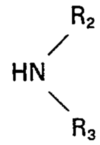

(VII)

in welcher $R_2$ und $R_3$ die oben angegebene Bedeutung haben, umsetzt.

5. Verfahren gemäß Anspruch 4, dadurch gekennzeichnet, daß man ein Verdünnungsmittel einsetzt.

6. Verfahren gemäß Anspruch 4, dadurch gekennzeichnet, daß die Umsetzungen bei Temperaturen von 20 bis 150°C durchgeführt werden.

7. Arzneimittel enthaltend mindestens eine Verbindung der Formel (I) gemäß Anspruch 1.

8. Verfahren zur Herstellung von Arzneimitteln, dadurch gekennzeichnet, daß man Verbindungen der Formel (I) gemäß Anspruch 1 gegebenenfalls unter Verwendung von üblichen Hilfs- und Trägerstoffen in eine geeignete Applikationsform überführt.

9. Verwendung von Verbindungen gemäß Anspruch 1 bei der Herstellung von Arzneimitteln zur Verbesserung der Herzkontraktilität, zur Erhöhung des Blutdruckes, zur Senkung von Blutzucker, zur Abschwellung von Schleimhäuten, zur Beeinflussung des Salz- und/oder Flüssigkeitshaushaltes.

**Patentansprüche für den Vertragsstaat: DE**

1. 1,4-Dihydropyridinamide der allgemeinen Formel I

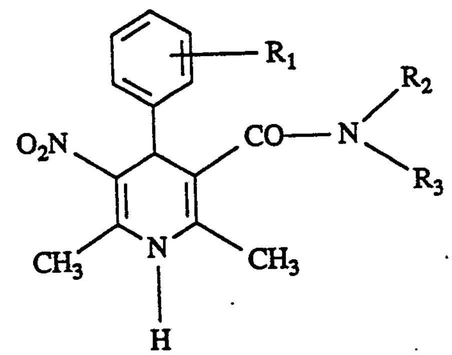

(I)

in welcher

$R_1$ Halogen, Trifluormethyl, Nitro, Wasserstoff oder eine der Gruppen

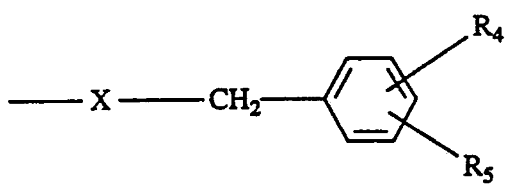

oder

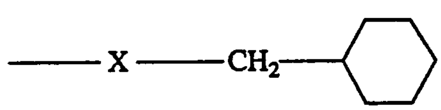

in denen X Sauerstoff oder Schwefel darstellt, wobei $R_4$ und $R_5$ gleich oder verschieden sein können und Wasserstoff, Halogen oder $C_1$—$C_4$-Alkyl bedeuten,

$R_2$ Wasserstoff oder $C_1$—$C_4$-Alkyl darstellt und

$R_3$ Wasserstoff, $C_2$—$C_4$-Alkenyl, $C_1$—$C_4$-Alkyl gegebenenfalls ein oder mehrfach substituiert durch Hydroxy, $C_3$—$C_6$-Cycloalkyl, Di-$C_1$—$C_4$-alkylamino-$C_1$—$C_4$-alkyl, Phenyl, $C_1$—$C_4$-Alkyl substituiert durch Phenyl, oder einen Kohlenhydratrest bedeutet,

mit Ausnahme der Verbindung 1,4-Dihydro-2,6-dimethyl-3-nitro-4-[2-(4-methylbenzyloxy)-phenyl]-5-carbonsäure-cyclopropylamid, sowie ihre pharmazeutisch unbedenklichen Additionssalze.

2. Verbindungen gemäß Anspruch 1 zur Bekämpfung von Kreislauferkrankungen.

3. Verbindungen gemäß Anspruch 1 zur Verbesserung der Herzkontraktilität, zur Erhöhung des

Blutdruckes, zur Senkung des Blutzuckers, zur Abschwellung von Schleimhäuten und/oder zur Beeinflussung des Salz- und Flüssigkeitshaushaltes.

4. Verfahren zur Herstellung von Verbindungen gemäß Anspruch 1, dadurch gekennzeichnet, daß man

A) Aminocrotonsäureamide der allgemeinen Formel (II)

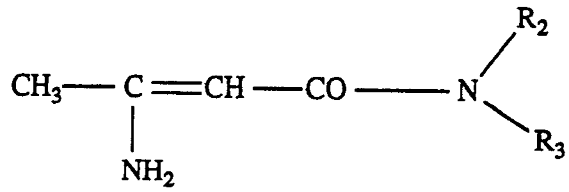

(II)

in welcher $R_2$ und $R_3$ die oben angegebene Bedeutung haben, mit Aldehyden der allgemeinen Formel (III)

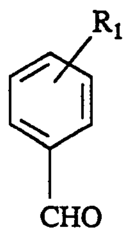

(III)

in welcher $R_1$ die oben angegebene Bedeutung hat und Nitroaceton

$$CH_3—CO—CH_2—NO_2$$

umsetzt oder

B) Benzaldehyde der Formel III und Acetessigsäureamide der Formel (IV)

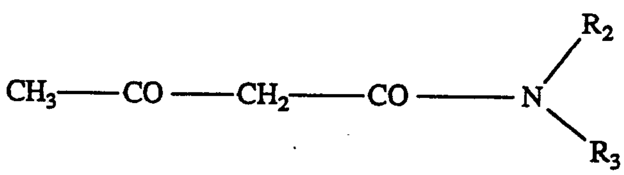

(IV)

in welcher $R_2$ und $R_3$ die oben angegebene Bedeutung haben bzw. deren Knoevenagel-Kondensationsprodukte der Formel (V)

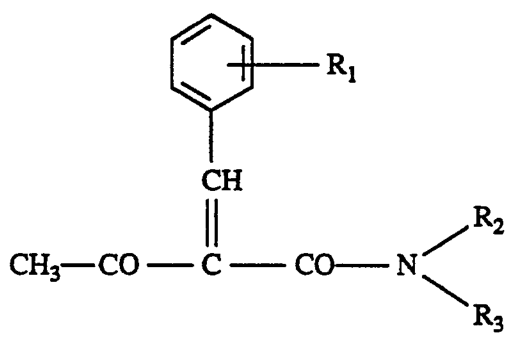

(V)

und einer Additionsverbindung aus Nitroaceton und Ammoniak

$$CH_3—CO—CH_2—NO_2 \cdot NH_3$$

umsetzt oder,

C) Dihydropyridine der Formel (VI)

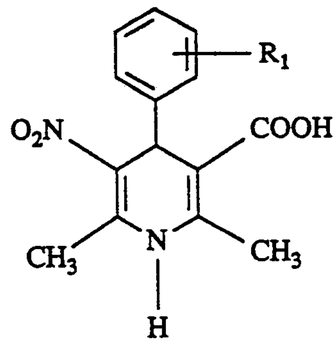

(VI)

in welcher $R_1$ die oben angegebene Bedeutung hat, nach an sich bekannten Methoden mit einem Amin umsetzt oder in ein reaktives Säurederivat überführt und dieses dann mit Aminen der allgemeinen Formel (VII)

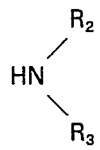

(VII)

in welcher $R_2$ und $R_3$ die oben angegebene Bedeutung haben, umsetzt.

5. Verfahren gemäß Anspruch 4, dadurch gekennzeichnet, daß man ein Verdünnungsmittel einsetzt.

6. Verfahren gemäß Anspruch 4, dadurch gekennzeichnet, daß die Umsetzungen bei Temperaturen von 20 bis 150°C durchgeführt werden.

7. Arzneimittel enthaltend mindestens eine Verbindung der Formel (I) gemäß Anspruch 1.

8. Verfahren zur Herstellung von Arzneimitteln, dadurch gekennzeichnet, daß man Verbindungen der Formel (I) gemäß Anspruch 1 gegebenenfalls unter Verwendung von üblichen Hilfs- und Trägerstoffen in eine geeignete Applikationsform überführt.

9. Verwendung von Verbindungen gemäß Anspruch 1 bei der Herstellung von Arzneimitteln zur Verbesserung der Herzkontraktilität, zur Erhöhung des Blutdruckes, zur Senkung von Blutzucker, zur Abschwellung von Schleimhäuten, zur Beeinflussung des Salz- und/oder Flüssigkeitshaushaltes.

**Revendications pour les Etats contractants: BE CH FR GB IT LI NL SE AT**

1. 1,4-dihydropyridinamides répondant à la formule générale (I)

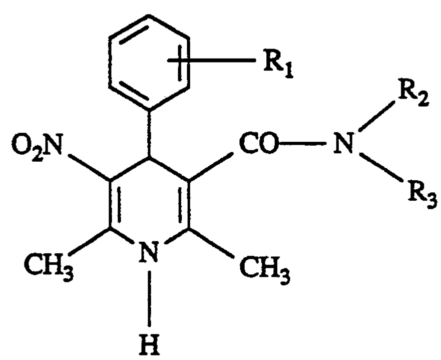

(I)

dans laquelle

$R_1$ représente un halogène, un radical trifluorométhyle, un radical nitro, de l'hydrogène ou l'un des groupes

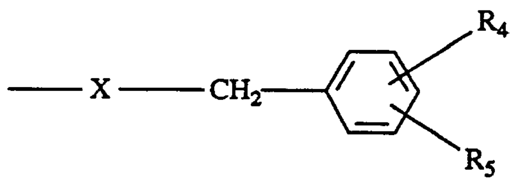

ou

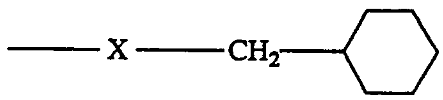

dans lesquels X est de l'oxygène ou du soufre tandis que $R_4$ et $R_5$ peuvent être identiques ou différents et représentent de l'hydrogène, un halogène ou un radical alkyle en $C_1$—$C_4$.

$R_2$ représente de l'hydrogène ou un radical alkyle en $C_1$—$C_4$.

$R_3$ représente de l'hydrogène, un alcényle en $C_2$—$C_4$, un alkyle en $C_1$—$C_4$ substitué éventuellement une ou plusieurs fois par un radical hydroxy, un radical cycloalkyle en $C_3$—$C_6$, un radical di-$C_1$—$C_4$-alkylamino-$C_1$—$C_4$-alkyle, un radical phényle, un radical alkyle en $C_1$—$C_4$ substitué par un phényle ou un radical d'hydrate de carbone,
ainsi que leurs sels d'addition acceptables en pharmacie.

2. Composés selon la revendication 1 pour lutter contre les affections du système circulatoire.

3. Composés selon la revendication 1, pour améliorer la contractilité du coeur, pour augmenter la pression sanguine, pour diminuer le sucre du sang, pour combattre le gonflement des muqueuses et/ou pour influencer le métabolisme des sels et des liquides.

4. Procédé de production des 1,4-dihydropyridinamides répondant à la formule générale I

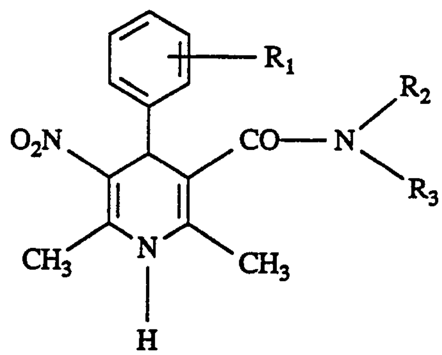

(I)

dans laquelle

$R_1$ représente un halogène, un radical trifluorométhyle, un radical nitro, de l'hydrogène ou l'un des groupes

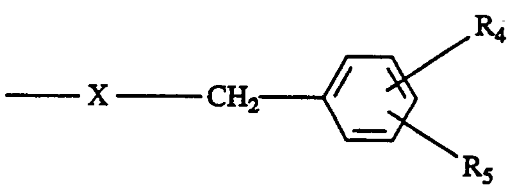

ou

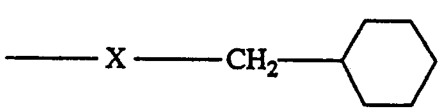

dans lesquels X représente de l'oxygène ou du soufre tandis que $R_4$ et $R_5$ peuvent être identiques ou différents et représentent de l'hydrogène, un halogène ou un radical alkyle en $C_1$—$C_4$,

$R_2$ représente de l'hydrogène ou un radical alkyle en $C_1$—$C_4$ et

$R_3$ représente de l'hydrogène, un radical alcényle en $C_1$—$C_4$, un radical alkyle $C_1$—$C_4$ substitué éventuellement une ou plusieurs fois par un radical hydroxy, un radical cycloalkyle en $C_3$—$C_6$, un radical di-$C_1$—$C_4$ alkylamino-$C_1$—$C_4$-alkyle, un radical phényle, un radical alkyle en $C_1$—$C_4$ substitué par un phényle ou un radical d'hydrate de carbone,
caractérisé en ce que l'on fait réagir

A) un amide de l'acide aminocrotonique répondant à la formule générale (II)

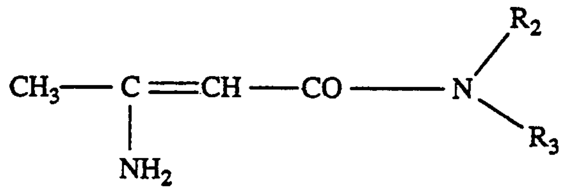

(II)

dans laquelle $R_2$ et $R_3$ ont les significations indiquées ci-dessus, avec des aldéhydes répondant à la formule générale (III)

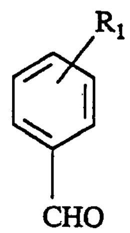

(III)

dans laquelle $R_1$ a la signification indiquée ci-dessus et avec de la nitroacétone

$$CH_3—CO—CH_2—NO_2$$

ou

B) on fait réagir du benzaldéhyde de la formule (III) et de l'amide d'acide acétoacétique de la formule (IV)

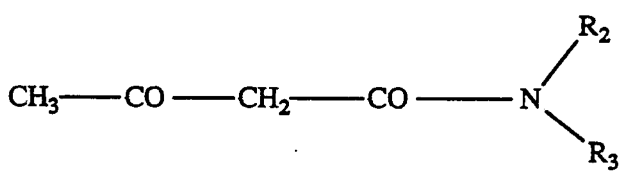

(IV)

dans laquelle $R_2$ et $R_3$ ont la signification mentionnée ci-dessus ou leurs produits de la condensation de Knoevenagel suivant la formule (V)

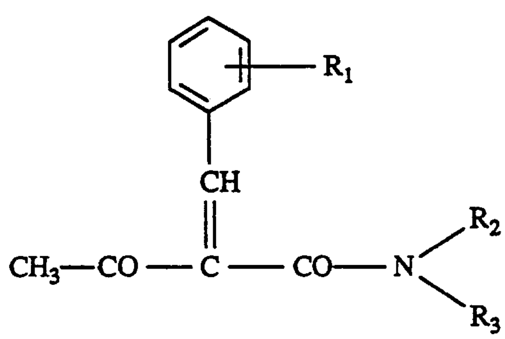

(V)

et un composé d'addition de la nitroacétone et de l'ammoniac

$$CH_3—CO—CH_2—NO_2 \cdot NH_3$$

ou

C) on fait réagir de la dihydropyridine répondant à la formule (VI)

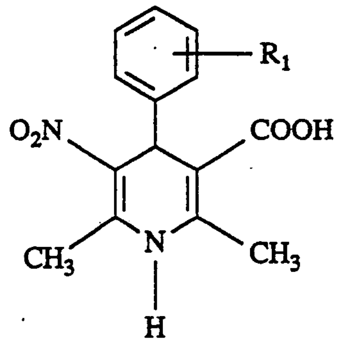

(VI)

dans laquelle $R_1$ a la signification indiquée ci-dessus avec une amine suivant des procédés connus en soi ou on la transforme en un dérivé d'acide réactif et que l'on fait réagir alors celui-ci avec une amine répondant à la formule générale (VII)

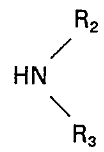

(VII)

dans laquelle $R_2$ et $R_3$ ont la signification indiquée ci-dessus.

5. Procédé selon la revendication 4, caractérisé en ce que l'on utilise un agent de dilution.

6. Procédé selon la revendication 4, caractérisé en ce que l'on effectue les réactions à des températures de 20 à 150°C.

7. Médicament contenant au moins un composé de la formule (I) selon la revendication 1.

8. Procédé de production de médicaments, caractérisé en ce que l'on transforme des composés de la formule (I) selon la revendication 1, éventuellement en utilisant des substances de support et auxiliaires usuelles en une forme d'administration appropriée.

9. Utilisation des composés selon la revendication 1 pour la production des médicaments pour améliorer la contractilité du coeur, pour augmenter la pression sanguine, pour diminuer le sucre sanguin, pour combattre le gonflement des muqueuses, pour influencer le métabolisme des sels et/ou des liquides.

**Revendications pour l'Etat contractant: DE**

1. 1,4-dihydropyridinamides répondant à la formule générale (I)

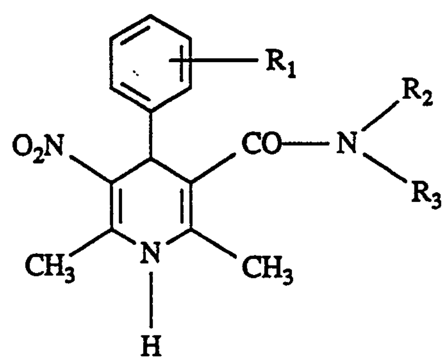

(I)

dans laquelle

$R_1$ représente un halogène, un radical trifluorométhyle, un radical nitro, de l'hydrogène ou l'un des groupes

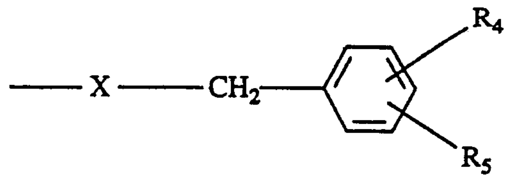

ou

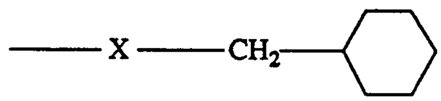

dans lesquels X est de l'oxygène ou du soufre tandis que $R_4$ et $R_5$ peuvent être identiques ou différents et représentent de l'hydrogène, un halogène ou un radical alkyle en $C_1$—$C_4$.

$R_2$ représente de l'hydrogène ou un radical alkyle en $C_1$—$C_4$.

$R_3$ représente de l'hydrogène, un alcényle en $C_2$—$C_4$, un alkyle en $C_1$—$C_4$ substitué éventuellement une ou plusieurs fois par un radical hydroxy, un radical cycloalkyle en $C_3$—$C_6$, un radical di-$C_1$—$C_4$-alkylamino-$C_1$—$C_4$-alkyle, un radical phényle, un radical alkyle en $C_1$—$C_4$ substitué par un phényle ou un radical d'hydrate de carbone,

à l'exception du composé cyclopropylamide de l'acide 1,4-dihydro-2,6-diméthyl-3-nitro-4-[2-(4-méthylbenzyloxy)-phényl]-5-carboxylique, ainsi que leurs sels d'addition acceptables en pharmacie.

2. Composés selon la revendication 1 pour lutter contre les affections du système circulatoire.

3. Composés selon la revendication 1, pour améliorer la contractilité du coeur, pour augmenter la pression sanguine, pour diminuer le sucre du sang, pour combattre le gonflement des muqueuses et/ou pour influencer le métabolisme des sels et des liquides.

4. Procédé de production des composés selon la revendication 1, caractérisé en ce que l'on fait réagir

A) un amide de l'acide aminocrotonique répondant à la formule générale (II)

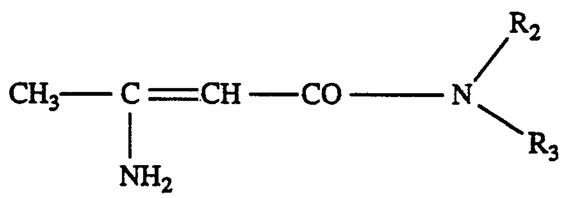

(II)

dans laquelle $R_2$ et $R_3$ ont les significations indiquées ci-dessus, avec des aldéhydes répondant à la formule générale (III)

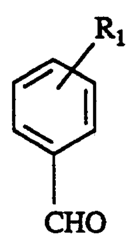

(III)

dans laquelle $R_1$ a la signification indiquée ci-dessus et avec de la nitroacétone

$$CH_3—CO—CH_2—NO_2$$

ou

B) on fait réagir du benzaldéhyde de la formule (III) et de l'amide d'acide acétoacétique de la formule (IV)

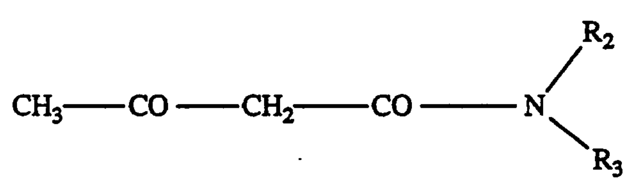

(IV)

dans laquelle $R_2$ et $R_3$ ont la signification mentionnée ci-dessus ou leurs produits de la condensation de Knoevenagel suivant la formule (V)

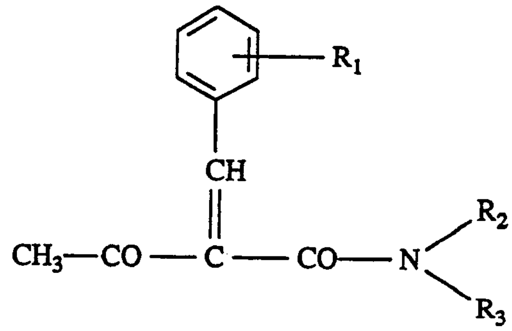

(V)

et un composé d'addition de la nitroacétone et de l'ammoniac

$$CH_3—CO—CH_2—NO_2 \cdot NH_3$$

ou

C) on fait réagir de la dihydropyridine répondant à la formule (VI)

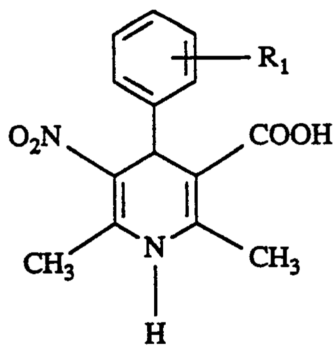

(VI)

dans laquelle $R_1$ a la signification indiquée ci-dessus avec une amine suivant des procédés connus en soi ou on la transforme en un dérivé d'acide réactif et que l'on fait réagir alors celui-ci avec une amine répondant à la formule générale (VII)

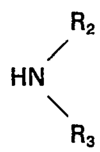

(VII)

dans laquelle $R_2$ et $R_3$ ont la signification indiquée ci-dessus.

5. Procédé selon la revendication 4, caractérisé en ce que l'on utilise un agent de dilution.

6. Procédé selon la revendication 4, caractérisé en ce que l'on effectue les réactions à des températures de 20 à 150°C.

7. Médicament contenant au moins un composé de la formule (I) selon la revendication 1.

8. Procédé de production de médicaments, caractérisé en ce que l'on transforme des composés de la formule (I) selon la revendication 1, éventuellement en utilisant des substances de support et auxiliaires usuelles en une forme d'administration appropriée.

9. Utilisation des composés selon la revendication 1 pour la production des médicaments pour améliorer la contractilité du coeur, pour augmenter la pression sanguine, pour diminuer le sucre sanguin, pour combattre le gonflement des muqueuses, pour influencer le métabolisme des sels et/ou des liquides.

**Claims for the Contracting States: BE CH FR GB IT LI NL SE AT**

1. 1,4-Dihydropyridine amides of the general formula I

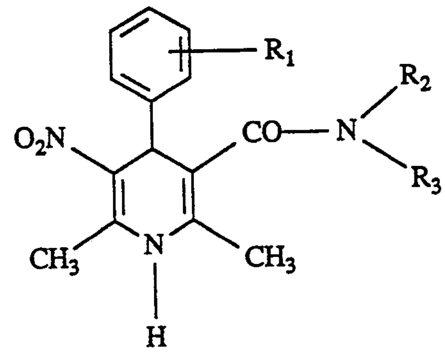

(I)

in which

$R_1$ represents halogen, trifluoromethyl, nitro, hydrogen or one of the groups

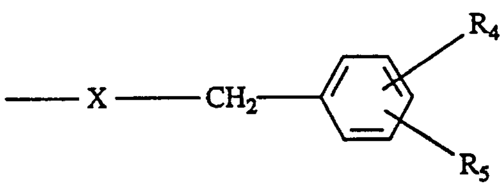

or

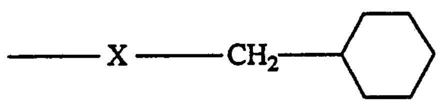

in which X represents oxygen or sulphur, where $R_4$ and $R_5$, which can be identical or different, denote hydrogen, halogen or $C_1$—$C_4$-alkyl,

$R_2$ represents hydrogen or $C_1$—$C_4$-alkyl and

$R_3$ denotes hydrogen, $C_2$—$C_4$-alkenyl, $C_1$—$C_4$-alkyl which is optionally monosubstituted or polysubstituted by hydroxyl, $C_3$—$C_6$-cycloalkyl, di-$C_1$—$C_4$-alkylamino-$C_1$—$C_4$-alkyl, phenyl, $C_1$—$C_4$-alkyl substituted by phenyl, or a carbohydrate radical,

and their pharmaceutically acceptable addition salts.

2. Compounds according to Claim 1 for controlling circulatory diseases.

3. Compounds according to Claim 1 for improving the contractility of the heart, for raising the blood pressure, for lowering the blood sugar, for reducing the swelling of mucous membranes and/or for affecting the salt and fluid balance.

4. Process for the preparation of 1,4-dihydropyridine amides of the general formula I,

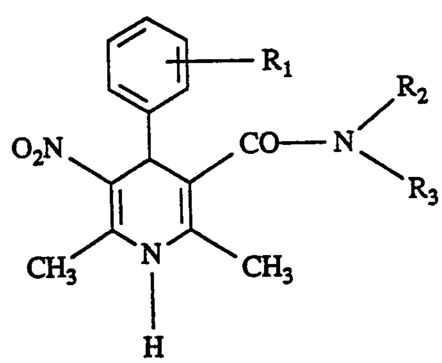

(I)

in which

$R_1$ represents halogen, trifluoromethyl, nitro, hydrogen or one of the groups

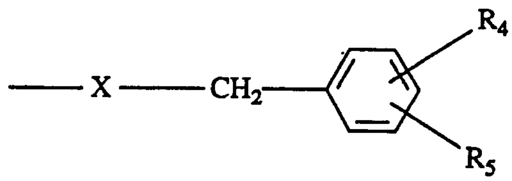

or

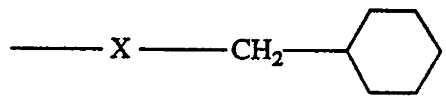

in which X represents oxygen or sulphur, where $R_4$ and $R_5$, which can be identical or different, denote hydrogen, halogen or $C_1$—$C_4$-alkyl,

$R_2$ represents hydrogen or $C_1$—$C_4$-alkyl and

$R_3$ denotes hydrogen, $C_2$—$C_4$-alkenyl, $C_1$—$C_4$-alkyl which is optionally monosubstituted or polysubstituted by hydroxyl, $C_3$—$C_6$-cycloalkyl, di-$C_1$—$C_4$-alkylamino-$C_1$—$C_4$-alkyl, phenyl, $C_1$—$C_4$-alkyl substituted by phenyl, or a carbohydrate radical,

characterized in that

A) amides of aminocrotonic acid of the general formula (II)

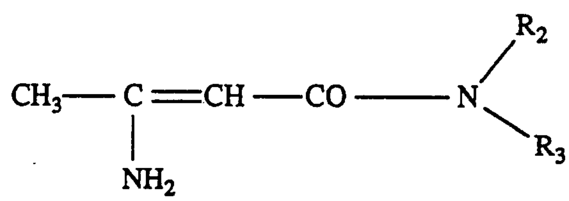

(II)

in which $R_2$ and $R_3$ have the meaning indicated above, are reacted with aldehydes of the general formula (III)

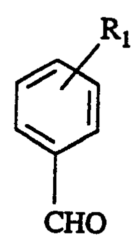

(III)

in which $R_1$ has the meaning indicated above and nitroacetone

$$CH_3—CO—CH_2—NO_2$$

or

B) benzaldehydes of the formula III are reacted with amides of acetoacetic acid of the formula (IV)

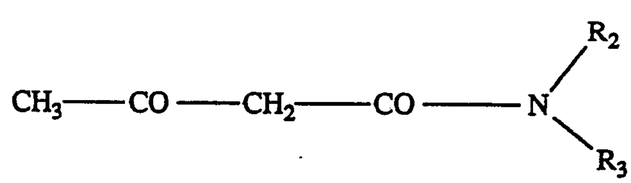

(IV)

in which $R_2$ and $R_3$ have the meaning indicated above or their Knoevenagel condensation products of the formula (V)

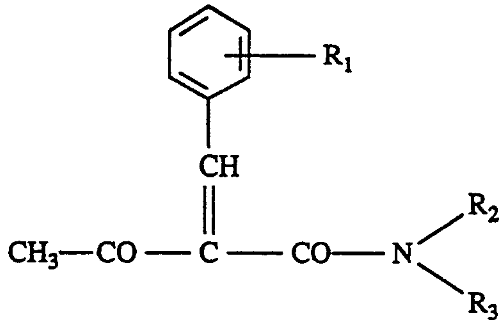

(V)

with an addition compound from nitroacetone and ammonia

$$CH_3—CO—CH_2—NO_2 \cdot NH_3$$

or

C) dihydropyridines of the formula (VI)

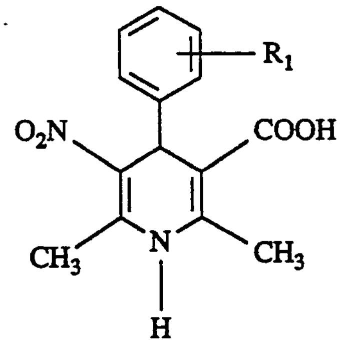

(VI)

in which $R_1$ has the meaning indicated above are reacted, by methods known per se, with an amine or are converted into a reactive acid derivative and the latter is then reacted with amines of the general formula (VII)

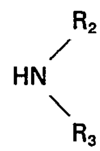

(VII)

in which $R_2$ and $R_3$ have the meaning indicated above.

5. Process according to Claim 4, characterized in that a diluent is employed.

6. Process according to Claim 4, characterized in that the reactions are carried out at temperatures of 20 to 150°C.

7. Medicament containing at least one compound of the formula (I) according to Claim 1.

8. Process for the preparation of medicaments, characterized in that compounds of the formula (I) according to Claim 1 are converted into a suitable form for administration, optionally with the use of customary auxiliaries and vehicles.

9. Use of compounds according to Claim 1 in the preparation of medicaments to improve the contractility of the heart, to raise the blood pressure, to lower the blood sugar, to reduce the swelling of mucous membranes, and to affect the salt and/or fluid balance.

**Claims for the Contracting State: DE**

1. 1,4-Dihydropyridine amides of the general formula

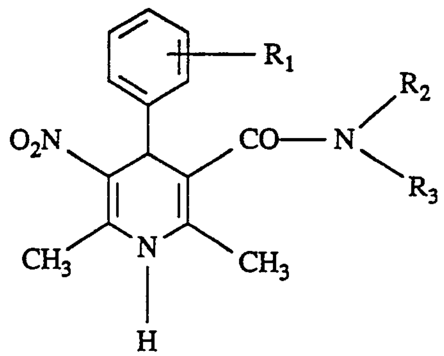

(I)

in which

$R_1$ represents halogen, trifluoromethyl, nitro, hydrogen or one of the groups

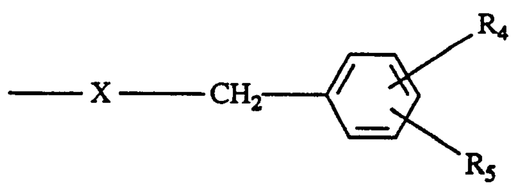

or

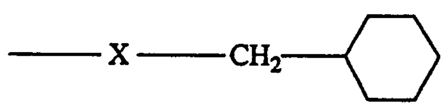

in which X represents oxygen or sulphur, where $R_4$ and $R_5$, which can be identical or different, denote hydrogen, halogen or $C_1$—$C_4$-alkyl,

$R_2$ represents hydrogen or $C_1$—$C_4$-alkyl and

$R_3$ denotes hydrogen, $C_2$—$C_4$-alkenyl, $C_1$—$C_4$-alkyl which is optionally monosubstituted or polysubstituted by hydroxyl, $C_3$—$C_6$-cycloalkyl, di-$C_1$—$C_4$-alkylamino-$C_1$—$C_4$-alkyl, phenyl, $C_1$—$C_4$-alkyl substituted by phenyl, or a carbohydrate radical,

with the exception of the compound 1,4-dihydro-2,6-dimethyl-3-nitro-4-[2-(4-methylbenzyloxy)phenyl]-5-carboxylic acid cyclopropylamide, and their pharmaceutically acceptable addition salts.

2. Compounds according to Claim 1 for controlling circulatory diseases.

3. Compounds according to Claim 1 for improving the contractility of the heart, for raising the blood pressure, for lowering the blood sugar, for reducing the swelling of mucous membranes and/or for affecting the salt and fluid balance.

4. Process for the preparation of compounds according to Claim 1, characterized in that

A) amides of aminocrotonic acid of the general formula (II)

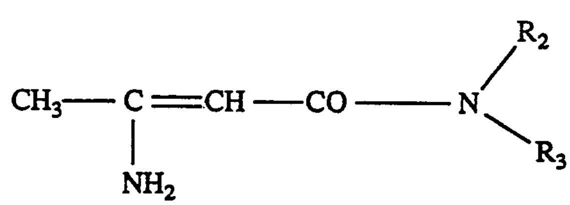

(II)

in which $R_2$ and $R_3$ have the meaning indicated above, are reacted with aldehydes of the general formula (III)

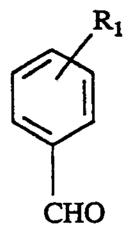

(III)

in which $R_1$ has the meaning indicated above and nitroacetone

$$CH_3—CO—CH_2—NO_2$$

or

B) benzaldehydes of the formula III are reacted with amides of acetoacetic acid of the formula (IV)

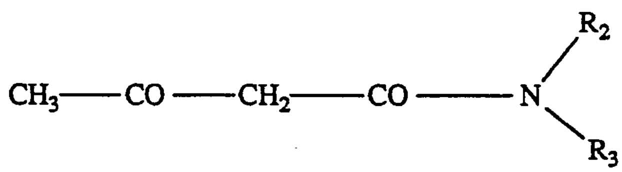

(IV)

in which $R_2$ and $R_3$ have the meaning indicated above or their Knoevenagel condensation products of the formula (V)

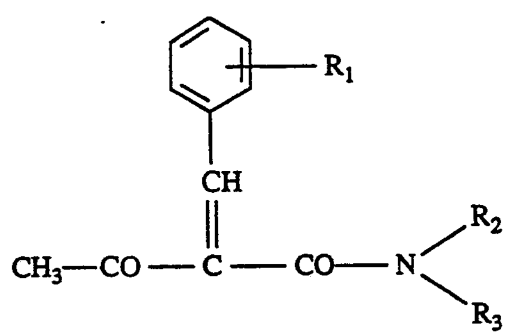

(V)

with an addition compound from nitroacetone and ammonia

$$CH_3—CO—CH_2—NO_2 \cdot NH_3$$

or

C) dihydropyridines of the formula (VI)

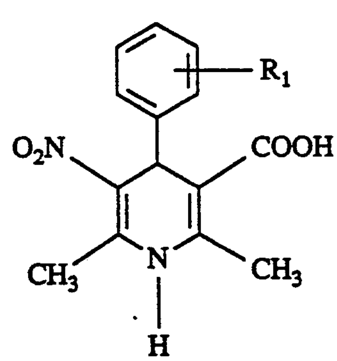

(VI)

in which $R_1$ has the meaning indicated above are reacted, by methods known per se, with an amine or are

converted into a reactive acid derivative and the latter is then reacted with amines of the general formula (VII)

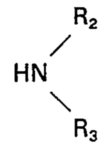

(VII)

in which $R_2$ and $R_3$ have the meaning indicated above.

5. Process according to Claim 4, characterized in that a diluent is employed.

6. Process according to Claim 4, characterized in that the reactions are carried out at temperatures of 20 to 150°C.

7. Medicament containing at least one compound of the formula (I) according to Claim 1.

8. Process for the preparation of medicaments, characterized in that compounds of the formula (I) according to Claim 1 are converted into a suitable form for administration, optionally with the use of customary auxiliaries and vehicles.

9. Use of compounds according to Claim 1 in the preparation of medicaments to improve the contractility of the heart, to raise the blood pressure, to lower the blood sugar, to reduce the swelling of mucous membranes, and to affect the salt and/or fluid balance.